(19) Europäisches Patentamt
European Patent Office
Office européen des brevets

(11) EP 0 894 805 B1

(12) **FASCICULE DE BREVET EUROPEEN**

(45) Date de publication et mention de la délivrance du brevet: **09.02.2005 Bulletin 2005/06**

(51) Int Cl.[7]: **C07H 17/075**, C07D 311/06, A61K 31/70

(21) Numéro de dépôt: **98401840.8**

(22) Date de dépôt: **21.07.1998**

(54) **Nouveaux dérivés aromatiques substitués par un ribose, leur procédé de préparation et leur application comme médicaments**

Ribosesubstituierte aromatische-Derivate, ihre Herstellung und ihre Verwendung als Arzneimitteln

Ribose substituted aromatic derivatives, their preparation and use as medicaments

(84) Etats contractants désignés:
**AT BE CH CY DE DK ES FI FR GB GR IE IT LI LU NL PT SE**

(30) Priorité: **23.07.1997 FR 9709352**

(43) Date de publication de la demande: **03.02.1999 Bulletin 1999/05**

(73) Titulaire: **Aventis Pharma S.A.**
**92160 Antony (FR)**

(72) Inventeurs:
- **Chartreaux, Fabienne**
  **93330 Neuilly sur Marne (FR)**
- **Klich, Michel**
  **93250 Villemomble (FR)**
- **Schio, Laurent**
  **93130 Noisy le Sec (FR)**

(56) Documents cités:
**CH-A- 467 799**
**US-A- 3 706 729**
**US-A- 5 412 104**

- **N.A.GORMLEY ET AL.: "The Interaction of Coumarin Antibiotics with Fragments of DNA Gyrase B Protein." BIOCHEMISTRY, vol. 35, no. 15, 16 avril 1996, pages 5083-5092, XP002058225**
- **D.R.BUCKLE ET AL.: "Aryloxyalkyloxy- and Aralkyloxy-4-hydroxy-3-nitrocoumarins Which Inhibit Histamine Release in the Rat and Also Antagonize the Effects of a Slow Reacting Substance of Anaphylaxis." JOURNAL OF MEDICINAL CHEMISTRY, vol. 22, no. 2, février 1979, pages 158-168, XP002058226**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Jouve, 75001 PARIS (FR)

## Description

La présente invention concerne de nouveaux dérivés aromatiques substitués par un ribose, leur procédé de préparation et leur application comme médicaments antibiotiques. Le document US 3,706,729 décrit des antibiotiques de la famille de la coumermycine qui ont une activité sur des bactéries gram® et gram®.

L'invention a pour objet les composés de formule (I) :

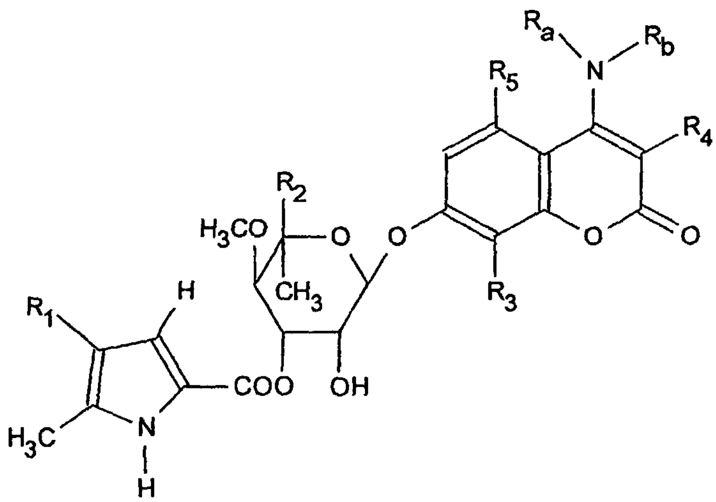

(I)

dans lesquels :

- $R_1$ représente un atome d'hydrogène ou d'halogène,
- $R_2$ représente un atome d'hydrogène ou un radical alkyle renfermant jusqu'à 4 atomes de carbone,
- $R_3$ représente un radical alkyle renfermant jusqu'à 4 atomes de carbone, ou un atome d'halogène,
- $R_4$ représente un atome d'hydrogène, un atome d'halogène, un radical alkyle, alkényle ou alkynyle renfermant jusqu'à 12 atomes de carbone, linéaire, ramifié ou cyclique,
- $R_5$ représente un atome d'hydrogène, un radical OH ou O-alkyle, renfermant jusqu'à 12 atomes de carbone,
- $R_a$ et $R_b$ identiques ou différents représentent un atome d'hydrogène, un radical alkyle renfermant jusqu'à 4 atomes de carbone, ou bien $R_a$ et $R_b$ forment avec l'atome d'azote auquel ils sont liés un hétérocycle mono ou polycyclique, renfermant éventuellement un autre hétéroatome choisi parmi l'azote, le soufre ou l'oxygène, ou bien $R_a$ et/ou $R_b$ représentent un radical :

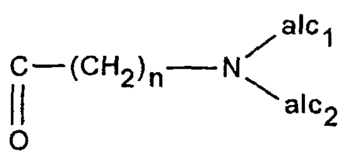

dans lequel n est un nombre entier variant de 0 à 6, $alc_1$ et $alc_2$ représentent un radical alkyle renfermant jusqu'à 8 atomes de carbone et leurs sels sous toutes leurs formes stéréoisomères possibles ainsi que leurs mélanges.

Comme exemples de sels on peut également citer les sels formés avec les acides acétique, propionique, trifluoroacétique, maléique, tartrique, méthanesulfonique, benzènesulfonique, p-toluènesulfonique, chlorhydrique, bromhydrique, iodhydrique, sulfurique, phosphorique et spécialement les acides stéariques, éthylsuccinique ou laurylsulfonique.

Dans la définition des substituants :

- le radical alkyle, alkényle ou alkynyle est de préférence un radical méthyle, éthyle, propyle, isopropyle, n-butyle, isobutyle, terbutyle, décyle ou dodécyle, vinyle, allyle, éthynyle, propynyle, cyclobutyle, cyclopentyle ou cyclohexyle,
- l'halogène est de préférence le fluor ou le chlore, ou le brome,

- le radical hétérocyclique est de préférence le radical pyrrolyle, pyrrolidinyle, pyridyle, pyrazinyle, pyrimidyle, pipéridinyle, pipérazinyle, quinuclidinyle, oxazolyle, isoxazolyle, morpholinyle, indolyle, imidazolyle, benzimidazolyle, triazolyle, thiazolyle, azétidinyle, aziridinyle.

Parmi les composés préférés de l'invention, on peut citer tout particulièrement les composés de formule (I) dans lesquels $R_1$ représente un atome d'hydrogène ou de fluor, ceux dans lesquels $R_2$ représente un radical méthyle ou un atome d'hydrogène, ceux dans lesquels $R_3$ représente un radical méthyle, ou un atome de chlore, ceux dans lesquels $R_4$ représente un atome d'hydrogène ou de chlore, ceux dans lesquel $R_5$ représente un atome d'hydrogène, ceux dans lesquels $R_a$ et $R_b$ représentent un radical alkyle renfermant jusqu'à 4 atomes de carbone, et notamment un radical méthyle, ceux dans lesquels $R_a$ et $R_b$ forment avec l'atome d'azote auquel ils sont liés un radical hétérocyclique renfermant éventuellement un autre hétéroatome, et pouvant être éventuellement substitué, et notamment ceux dans lesquels $R_a$ et $R_b$ forment ensemble un radical

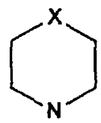

dans lequel X représente un atome d'oxygène ou un radical NH, ou $NCH_3$, le radical hétérocyclique ainsi formé pouvant être éventuellement substitué.

L'invention a plus particulièrement pour objet les composés dont la préparation est donnée ci-après dans la partie expérimentale et tout spécialement les composés des exemples 1 et 2.

Les produits de formule générale (I) possèdent une très bonne activité antibiotique sur les bactéries gram® telles que les staphylocoques, les streptocoques, les pneumocoques, les entérocoques, listeria, anaerobies.

Les composés de l'invention peuvent donc être utilisés comme médicaments dans le traitement des infections à germes sensibles et, notamment, dans celui des staphylococcies, telles que les septicémies à staphylocoques, staphylococcies malignes de la face ou cutanées, pyodermites, plaies septiques ou suppurantes, furoncles, anthrax, phlegmons, érysipèles et acné, staphylococcies telles que les angines aigües primitives ou post-grippales, bronchopneumonie, suppuration pulmonaires, les streptococcies telles que les angines aigües, les otites, les sinusites, la scarlatine, les pneumococcies telles que les pneumonies, les bronchites et la diphtérie. Les produits de la présente invention sont également actifs contre les infections dues à des germes comme Haemophilus influenzae.

L'invention a donc pour objet les composés de formule (I) à titre de médicaments antibiotiques.

L'invention a plus spécialement pour objet à titre de médicaments antibiotiques les composés indiqués ci-dessus comme composés préférés.

L'invention a également pour objet les compositions pharmaceutiques renfermant comme principe actif au moins un des médicaments antibiotiques définis ci-dessus.

Ces compositions peuvent être administrées par voie buccale, rectale, parentérale ou par voie locale en application topique sur la peau et les muqueuses, mais la voie d'administration préférée est la voie buccale ou injectable.

Elles peuvent être solides ou liquides et se présenter sous les formes pharmaceutiques couramment utilisées en médecine humaine, comme par exemple, les comprimés simples ou dragéifiés, les gélules, les granulés, les suppositoires, les préparations injectables, les pommades, les crèmes, les gels ; elles sont préparées selon les méthodes usuelles. Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

Ces compositions peuvent également se présenter sous forme d'une poudre destinée à être dissoute extemporanément dans un véhicule approprié, par exemple de l'eau stérile apyrogène.

La dose administrée est variable selon l'affection traitée, le sujet en cause, la voie d'administration et le produit considéré. Elle peut être, par exemple, comprise entre 50 mg et 3000 mg par jour par voie orale ou injectable, chez l'adulte pour les produits préférés.

L'invention a également pour objet un procédé caractérisé en ce que l'on soumet un composé de formule (II) :

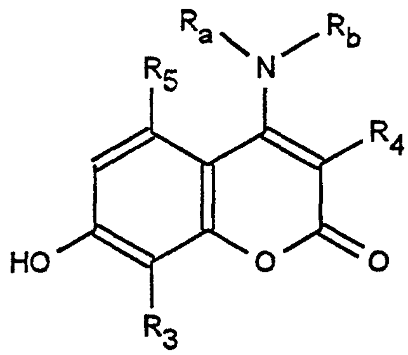

(II)

dans lequel $R_3$, $R_4$, $R_5$, $R_a$ et $R_b$ conservent leur signification précédente, à l'action d'un composé de formule (III) :

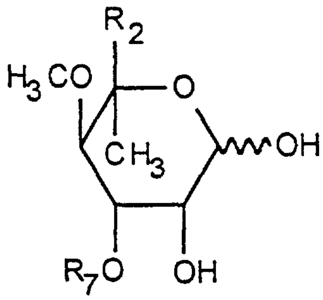

(III)

dans lequel $R_2$ conserve sa signification précédente et $R_7$ représente un radical

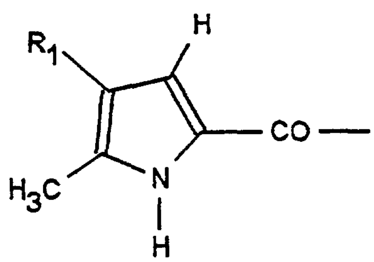

dans lequel $R_1$ conserve sa signification précédente, pour obtenir le composé de formule (I) correspondant que l'on modifie si désiré.

L'invention a également pour objet une variante du procédé caractérisée en ce que l'on soumet un composé de formule (IV) :

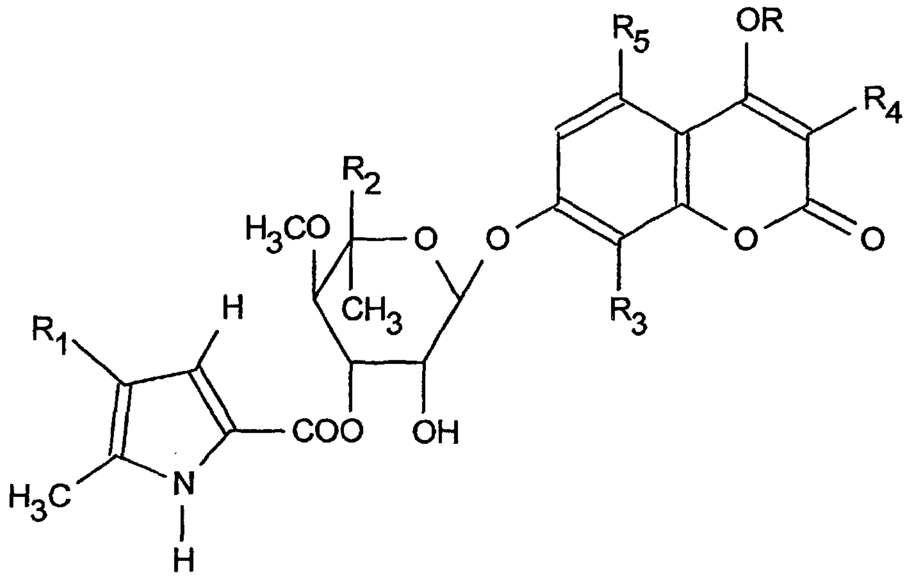

(IV)

dans lequel $R_1$, $R_2$, $R_3$, $R_4$ et $R_5$ conservent leur signification précédente et R représente un groupe partant à l'action d'une amine

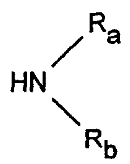

,

dans laquelle $R_a$ et $R_b$ conservent leur signification précédente pour obtenir le composé de formule (I) correspondant que l'on modifie si désiré.

Les composés de formules (II) et (IV) telles que définies ci-dessus, à l'exception du 4-amino 7-hydroxy-8-methyl-benzopyran-2-one, du 4-amino-8-ethyl-7-hydroxycoumarin et du 4-hydroxy-7α-3-O-[5'-methyl-2'-carbonyl-pyrrole]-noviosyloxy-8-methyl-coumarin,sont des produits nouveaux, leur préparation est donnée ci-après dans la partie expérimentale, et sont en eux-mêmes un objet de la présente invention.

Les composés de formule (II) peuvent être préparés selon les procédé décrits dans la partie expérimentale, par exemple selon le mode opératoire suivant :

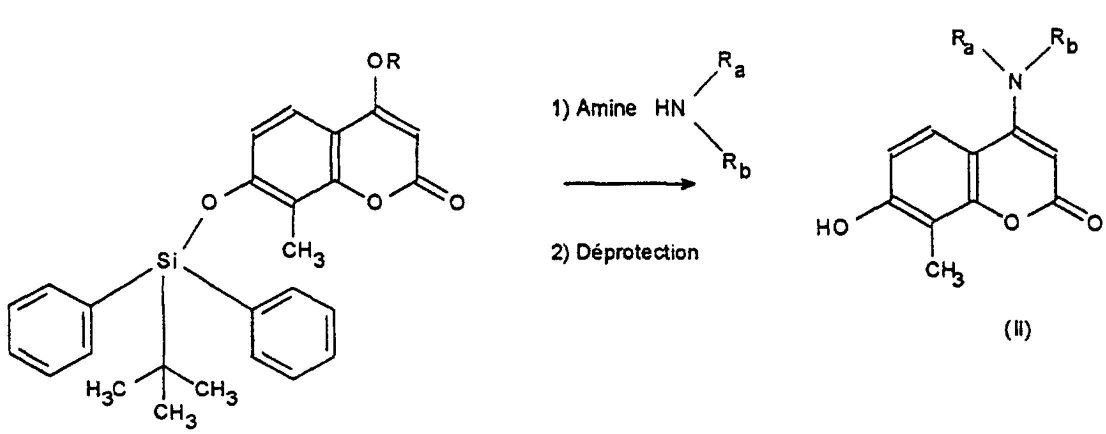


(II)

Comme groupes partant R on peut citer le reste d'un tosylate, d'un mésylate, d'un triflate.

Les composés de formule (IV) peuvent être préparés selon les procédés décrits dans la partie expérimentale :

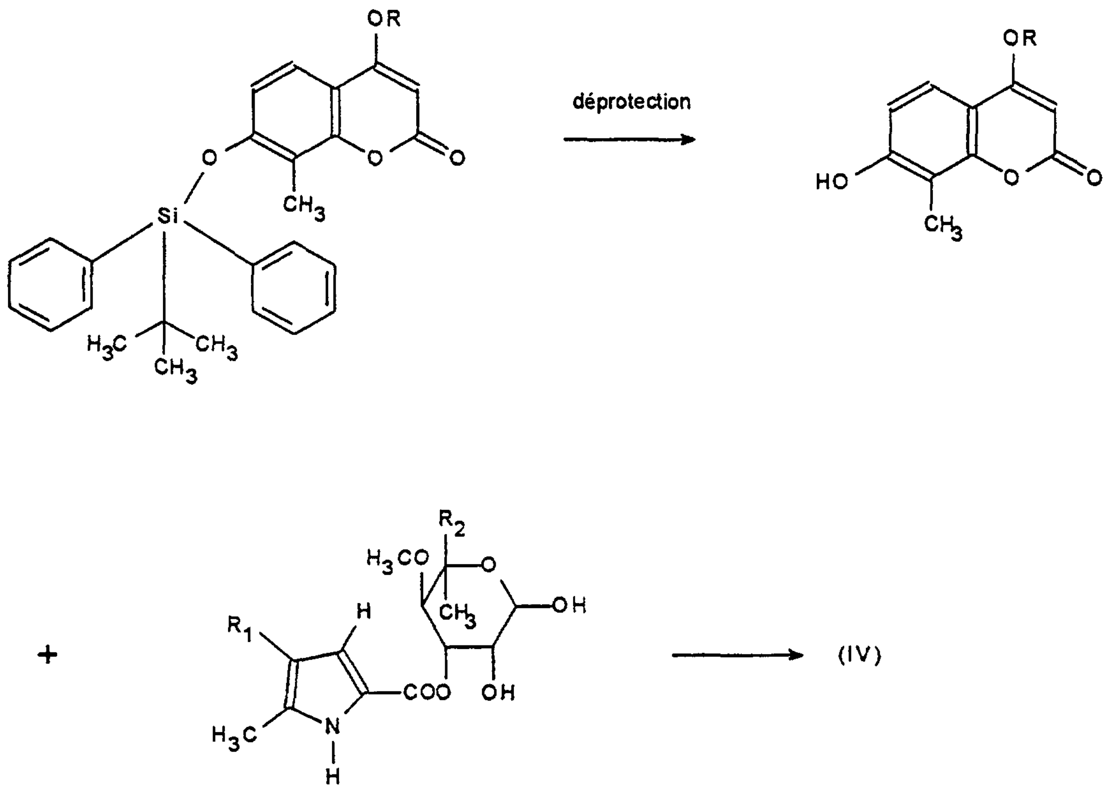


(IV)

Dans ce qui précède R a la même valeur que les groupes partants précédents.

**<u>EXEMPLE 1</u> : 6-déoxy-5-C-méthyl-4-O-méthyl-3-O-(5-méthyl-1H-pyrrol-2-yl) carbonyl) alpha-L-lyxo-hexopyranoside de (4-(diméthylamino)-8-méthyl-2-oxo-2H-1-benzopyran-7-yle)**

On introduit à 100°C, 5,3 ml d'une solution de chlorure de cobalt 0,1N dans l'acétonitrile, dans une solution renfermant 210 mg de 7-(6-déoxy-5-C-méthyl-4-O-méthyl-alpha-L-lyxohexopyranosyl) oxy-4-(diméthylamino)-8-méthyl-2H-1-benzopyran-2-one et 124 mg d'anhydride pyrrolique. On agite à 100°C pendant 15 minutes, laisse refroidir et concentre sous pression réduite. On obtient un produit que l'on purifie par chromatographie sur silice en éluant avec le mélange chlorure de méthylène-acétone (80-20). On obtient 56,2 mg de produit recherché.
rf = 0,25, F > 300°C.

**<u>PREPARATION</u> : 7-[(6-déoxy-5-C-méthyl-4-O-méthyl-alpha-L-lyxo-hexopyranosyl) oxy]-4-(diméthylamino)-8-méthyl-2H-1-benzopyran-2-one**

<u>STADE A</u> : 7-[[(1,1-diméthyléthyl)diphénylsilyl]oxy]-4-hydroxy-8-méthyl-2H-1-benzopyran-2-one

On agite 85 ml de triéthylamine dans une suspension renfermant 50 g de 4,7-dihydroxy-8-méthyl-2H-1-benzopyran-2-one et 400 ml de tétrahydrofuranne. On ajoute une solution de 76 ml du terbutyldiphénylchlorosilane dans 50 ml de tétrahydrofuranne. On agite le mélange réactionnel pendant 20 heures. On verse sur une solution tampon d'hydrogéno phosphate de sodium. On extrait à l'acétate d'éthyle, lave la phase organique à l'eau, sèche et amène à sec. On obtient un produit que l'on reprend dans le méthanol, on agite pendant 4 heures, essore, lave et sèche. On obtient 84,5 g de produit recherché.

<u>STADE B</u> : 7-[[(1,1-diméthyléthyl)diphénylsilyl]oxy]-8-méthyl-4-[[(trifluorométhyl)sulfonyl]oxy]-2H-1-benzopyran-2-one

On introduit à 0°C, dans une suspension de 15 g du produit du stade A dans 150 ml de chlorure de méthylène, 7,3 ml de triéthylamine. On ajoute une solution de 7,1 ml d'anhydride trifluorométhyl sulfonique et 7 ml de chlorure de

méthylène. On maintient le mélange réactionnel sous agitation pendant 30 minutes à 0°C, puis 1 heure à la température ambiante. On verse dans 150 ml d'une solution 1M de phosphate acide de sodium, extrait au chlorure de méthylène, lave, sèche et concentre sous pression réduite. On obtient un produit que l'on chromatographie sur silice en éluant avec le mélange hexane-acétate d'éthyle (8-2). On obtient ainsi 17,1 g du produit recherché. rf = 0,49.

STADE C : 4-(diméthylamino)-7-hydroxy-8-méthyl-2H-1-benzopyran-2-one

Dans une solution de 1,05 g de produit préparé au stade B et 10 ml de THF, on introduit 11,3 ml d'une solution 0,6 M de diméthylamine dans le THF. On maintient sous agitation pendant une nuit. On fait barboter de l'azote, refroidit la réaction à l'aide d'un bain de glace puis coule 1,9 ml de fluorure de n-tétrabutylammonium (solution 1,1 M dans le THF). On agite pendant 15 minutes à 0°C. On verse dans 20 ml d'une solution 1M de phosphate acide de sodium. On extrait au chlorure de méthylène. On lave les phases organiques, sèche et concentre. On obtient un produit que l'on reprend dans la méthyléthylcétone. On essore, lave à la méthyléthylcétone et sèche. On concentre le filtrat, reprend dans l'acétate d'éthyle, essore et sèche. On obtient 350,4 mg de produit recherché. rf = 0,18 chlorure de méthylène/ acétone (9-1).

STADE D : 7-[(6-déoxy-5-C-méthyl-4-O-méthyl-alpha-L-lyxo-hexopyranosyl) oxy]-4-(diméthylamino)-8-méthyl-2H-1-benzopyran-2-one

On introduit 1,2 g de triphénylphosphine et 796 µl de DEAD (diéthylazodicarboxylate) dans une suspension renfermant 830 mg du produit du stade précédent et 804 mg de 6-déoxy-5-C-méthyl-4-O-méthyl-alpha-L-lyxo-hexopyranose et 20 ml de chlorure de méthylène. On agite une heure à la température ambiante et effectue deux additions à une heure d'intervalle de 0,6 puis 0,4 équivalents de triphényl phosphine et DEAD. On concentre et chromatographie sur silice en éluant avec le mélange chlorure de méthylène/acétone (80-20). On obtient après traitement à l'acétone le produit recherché rf = 0,29 (chlorure de méthylène-acétone (6-4)).

**EXEMPLE 2 :6-déoxy-5-C-méthyl-4-O-méthyl-3-O-(5-méthyl-1H-pyrrol-2-yl) carbonyl) alpha-L-lyxo-hexopyranoside de (8-méthyl-4-(4-méthyl-1-pipérazinyl)-2-oxo-2H-1-benzopyran-7-yle)**

On ajoute 354 mg de triphénylphosphine et 224 µl de DEAD dans une dispersion renfermant 500 mg de 7-hydroxy-8-méthyl-4-(4-méthyl-1-pipérazinyl)-2H-1-benzopyran-2-one et 370 mg de 3-(5-méthyl-1H-pyrrole-2-carboxylate) de 6-déoxy-5-C-méthyl-4-O-méthyl-L-lyxohexopyranose. Après 2 heures de réaction, on ajoute 354 mg de triphénylphosphine et 224 µl de DEAD. On agite pendant 2 heures et ajoute 283 mg de triphénylphosphine et 180 µl de DEAD. On agite encore pendant 2 heures, et ajoute 142 mg de triphénylphosphine et 90 µl de DEAD. On agite pendant 2 heures, concentre et chromatographie sur silice, éluant MeOH-$CH_2Cl_2$ (5-95). rf = 0,21. On isole ainsi 373 mg de produit recherché. F = 150°C.

**PREPARATION : 7-hydroxy-8-méthyl-4-(4-méthyl-1-pipérazinyl)-2H-1-benzopyran-2-one**

STADE A : 7-[[(1,1-diméthyléthyl)diphénylsilyl]oxy]-8-méthyl-4-(4-méthyl-1-pipérazinyl)-2H-1-benzopyran-2-one

On ajoute 1,25 ml de N-méthyl pipérazine dans une solution renfermant 2,53 g de 7-[[(1,1-diméthyléthyl) diphénylsilyl]oxy]-8-méthyl-4-[[(trifluorométhyl)sulfonyl]oxy]-2H-1-benzopyran-2-one et 22 ml de THF. On maintient sous agitation pendant 1 heure. On verse sur une solution aqueuse 1M de phosphate acide de sodium, et extrait à l'acétate d'éthyle. On réunit les phases organiques, les lave, les sèche et les concentre sous pression réduite. On chromatographie sur silice en éluant avec le mélange méthanol-chlorure de méthylène (6-94). On isole 2,31 g de produit recherché. rf = 0,3.

STADE B : 7-hydroxy-8-méthyl-4-(4-méthyl-1-pipérazinyl)-2H-1-benzopyran-2-one

On ajoute à 0°C, 5,10 ml d'une solution 1,1M de fluorure de n-tétrabutyl ammonium dans le THF. On maintient sous agitation pendant 1 heure à 0°C et ajoute 270 µl d'acide acétique. On concentre et chromatographie sur silice en éluant avec le mélange méthanol-chlorure de méthylène 5-95 puis 10-90. On reprend au chlorure de méthylène, essore et isole 0,88 g de produit. rf = 0,26 méthanol-chlorure de méthylène 10-90.

**EXEMPLE 3 : iodure de 6-déoxy-5-C-méthyl-4-O-méthyl-3-O-(5-méthyl-1H-pyrrol-2-yl) carbonyl) alpha-L-lyxo-hexopyranoside de (4-(4,4-diméthylpipérazinium)-8-méthyl-2-oxo-2H-1-benzopyran-7-yle)**

A une dispersion de 50 mg du produit de l'exemple précédent dans 1 ml d'acétonitrile, on ajoute 8,5 µl d'iodométhane. On maintient le mélange réactionnel obtenu sous agitation à 60°C, refroidit et concentre sous pression réduite. On reprend au chlorure de méthylène et essore. On obtient 52 mg de produit recherché. F = 230°C.

**EXEMPLE 4 : Acide 5-méthyl-1H-pyrrole-2-carboxylique-3'-ester de N-(7-((6-déoxy-5-C-méthyl-4-O-méthyl-alpha-L-lyxo-hexopyranosyl) oxy)-8-méthyl-2-oxo-2H-1-benzopyran-4-yl-2-(diméthylamino) acétamide**

On ajoute 152 mg de 2-(diméthylamino)-N-(7-hydroxy-8-méthyl-2-oxo-2H-1-benzopyran-4-yl) acétamide, 106 mg de triphénylphosphine et 67 µl de DEAD dans une dispersion renfermant 118 mg d'acide 5-méthyl-1H-pyrrole-2-carboxylique ester de 6-déoxy-5-C-méthyl-4-O-méthyl-alpha-L-lyxo-hexopyranose et 3,5 ml de chlorure de méthylène. On maintient sous agitation pendant 2 heures à la température ambiante et ajoute à nouveau 106 mg de triphénylphosphine et 67 µl de DEAD. Après 2 heures de réaction supplémentaires, on ajoute à nouveau 42 mg de triphénylphosphine et 27 µl de DEAD. On maintient l'agitation pendant 2 heures et chromatographie le produit obtenu, d'abord en éluant avec le mélange méthanol-chlorure de méthylène (5-95) puis avec le mélange acétone-chlorure de méthylène (20-80). On isole ainsi 72 mg du produit recherché. F = 260°C.

**PREPARATION : 2-(diméthylamino)-N-(7-hydroxy-8-méthyl-2-oxo-2H-1-benzopyran-4-yl)acétamide**

STADE A : 4-amino-7-[[[(1,1-diméthyléthyl)diphénylsiyl]oxy]-8-méthyl-2-oxo-2H-1-benzopyran-4-yl]-acétamide

On ajoute 783 mg d'imidazole puis 1,43 ml de $tBuPh_2SiCl$ dans une dispersion renfermant 1 g de 4-amino-7-hydroxy-8-méthyl-2H-1-benzopyran-2-one et 25 ml de DMF anhydre. On agite le milieu réactionnel pendant 16 heures à température ambiante, verse dans une solution aqueuse (1M) de phosphate acide de sodium et extrait avec du THF. On décante la phase aqueuse et extrait à l'acétate d'éthyle. On réunit les phases organiques, les lave à l'eau, les sèche et concentre sous pression réduite. On chromatographie le produit obtenu sur silice en éluant avec le mélange méthanol-chlorure de méthylène (5-95). On reprend le produit obtenu à l'éther et essore. On obtient 1,01 g de produit recherché. rf = 0,33, méthanol-chlorure de méthylène (5-95).

STADE B : 2-chloro-N-[7-[[(1,1-diméthyléthyl) diphénylsilyl] oxy]-8-méthyl-2-oxo-2H-1-benzopyran-4-yl]-acétamide.

On ajoute à 0°C, 752 µl de TEA et 430 µl de chlorure de chloroacétyle dans une solution renfermant 885 mg du produit du stade précédent et 18 ml de THF. On agite pendant 38 heures et verse sur une solution aqueuse de phosphate acide de sodium (1M) et extrait deux fois à l'acétate d'éthyle. On réunit les phases organiques, les lave, sèche et concentre sous pression réduite. On chromatographie le produit obtenu sur silice en éluant avec le mélange acétate d'éthyle-chlorure de méthylène (2-98). On isole ainsi 453 mg de produit recherché.

STADE C : 2-(diméthylamino)-N-(7-hydroxy-8-méthyl-2-oxo-2H-1-benzopyran-4-yl)acétamide

On ajoute à 0°C quelques gouttes de diméthylamine dans une solution renfermant 351 mg du produit du stade précédent et 7 ml de THF. On maintient sous agitation pendant 2 heures à la température ambiante et concentre sous pression réduite. On empâte le résidu obtenu dans l'acétone et essore. On lave avec du chlorure de méthylène, puis avec du pentane. On sèche et isole 110 mg de produit. Le filtrat est chromatographié sur silice en éluant avec le mélange méthanol-chlorure de méthylène (5-95). On isole ainsi 32 mg supplémentaires de produit recherché rf = 0,27.

**EXEMPLE 5 : Acide 5-méthyl-1H-pyrrole-2-carboxylique-3'-ester de 7-(6-déoxy-5-C-méthyl-4-0-méthyl-alpha-L-lyxo-hexopyranosyl) oxy)-8-méthyl-4-(4-morpholinyl)-2H-1-benzopyran-2-one**

En opérant comme précédemment à partir de 387 mg de 7-hydroxy-8-méthyl-4-(4-morpholinyl)-2H-1-benzopyran-2-one et 487 mg de 3-(5-méthyl-1H-pyrrole-2-carboxylate) de 6-déoxy-5-C-méthyl-4-O-méthyl-L-lyxohexopyranose, on a obtenu 115,4 mg de produit recherché. F > 260°C.

**EXEMPLE 6 : Acide 4-fluoro-5-méthyl-1H-pyrrole-2- carboxylique-3'-ester de 7-((6-déoxy-5-C-méthyl-4-O-méthyl-alpha-L-lyxo-hexopyranosyl) oxy)-4-diméthylamino)-8-méthyl-2H-1-benzopyran-2-one**

On ajoute 213 mg d'acide 4-fluoro-5-méthyl-1H-pyrrole-2-carboxylique puis 182 mg de DMAP, puis 233 µl de

DIC dans une dispersion renfermant 585 mg de 7-[(6-déoxy-5-C-méthyl-4-O-méthyl-alpha-L-lyxo-hexopyranosyl) oxy]-4-(diméthylamino)-8-méthyl-2H-1-benzopyran-2-one et 15 ml de chlorure de méthylène. On agite pendant 14 heures à la température ambiante et ajoute 222 µl de DBU. On agite pendant 2 heures à la température ambiante puis chromatographie le produit obtenu en éluant avec le mélange méthanol-chlorure de méthylène (5-95). On reprend le produit obtenu dans l'acétone et essore. On reprend au méthanol et essore. On isole 77 mg de produit recherché.
F > 260°C.

**EXEMPLE 7 : Acide 5-méthyl-1H-pyrrole-2-carboxylique-3'-ester de 7-((6-déoxy-5-C-méthyl-4-O-méthyl-alpha-L-lyxo-hexopyranosyl) oxy)-4-(2-diméthylamino) éthylméthylamino)-8-méthyl-2H-1-benzopyran-2-one**

Le produit a été préparé en opérant comme précédemment, rf = 0,15, $CH_2Cl_2$-MeOH-$NH_3$ (92-8-0,5).

**EXEMPLE 8 : Acide 5-méthyl-1R-pyrrole-2-carboxylique-3'-ester de 7-((6-déoxy-5-C-méthyl-4-O-méthyl-alpha-L-lyxo-hexopyranosyl) oxy)-8-méthyl-4-(1-pipérazinyl)-2H-1-benzopyran-2-one**

F = 190~200°C.

**EXEMPLE 9 : 6-déoxy 5-méthyl-4-O-méthyl-3-O-((5-méthyl-1H-pyrrol-2-yl)carbonyl)-alpha-L-lyxo-hexopyranoside de (8-méthyl-4-(4-méthyl-1-pipérazinyl)-2-oxo-2H-1-(3-chlorobenzo pyran)-7-yle**

En opérant comme précédemment à partir du 3-chloro-7-hydroxy-8-méthyl-4-(4-méthyl-1-pipérazinyl)-2H-1-benzopyran-2-one, on a obtenu le produit recherché. F = 145°C.

**EXEMPLE 10 : Acide 5-méthyl-1H-pyrrole-2-carboxylique-3'-ester de 7-((6-déoxy-5-C-méthyl-4-O-méthyl-alpha-L-lyxo-hexopyranosyl) oxy)-4-(4-éthyl-1-pipérazinyl)-8-méthyl-2H-1-benzopyran-2-one**

On met en suspension dans 12 ml de chlorure de méthylène, 374 mg de 7-hydroxy 8-méthyl-4-(4-éthyl-1-pipérazinyl) 2H-1-benzopyran-2-one, 424 mg de 3-(5-méthyl-1H-pyrrole-2-carboxylate) de 6-déoxy-5-C-méthyl-4-O-méthyl-L-lyxohexopyranose et 408 mg de triphénylphosphine. On ajoute 275 µl de DEAD. On agite 2 heures à la température ambiante et ajoute 408 mg de triphénylphosphine et 275 µl de DEAD. On agite pendant 1 H 45 et ajoute 170 mg de triphénylphosphine et 113 µl de DEAD. On chromatographie le produit obtenu en éluant avec le mélange chlorure de méthylène-isopropanol (9-1). On verse le produit sur 30 ml d'une solution molaire de dihydrogénophosphate de sodium et extrait au chlorure de méthylène. On lave avec une solution saturée de chlorure de sodium, on réunit les phases organiques et les sèche sur sulfate de magnésium. On isole 2,61 g de produit que l'on chromatographie sur silice en éluant avec le mélange chlorure de méthylène-méthanol (90-10). On isole la fraction de rf = 0,10, on obtient 350 mg de produit que l'on empâte à l'acétonitrile, essore le produit obtenu, amène à sec le filtrat, reprend à l'acétonitrile, essore et joint à la première fraction obtenue. On isole ainsi 170 mg de produit recherché. F = 246~248°C.

**EXEMPLE 11 : Acide 5-méthyl-1H-pyrrole-2-carboxylique-3'-ester de 7-((6-déoxy-5-C-méthyl-4-O-méthyl-alpha-L-lyXo-hexopyranosyl) oxy)-8-méthyl-4-(4-(2-pyrimidyl)-1-pipérazinyl)-2H-1-benzopyran-2-one**

En opérant comme précédemment à partir de 7-hydroxy-8-méthyl-4-[4-(2-pyrimidinyl)-1-pipérazinyl]-2H-1-benzopyran-2-one, on a obtenu le produit recherché. rf = 0,25, chlorure de méthylène-isopropanol (97-3).
En opérant comme précédemment, on a obtenu les produits suivants :

**EXEMPLE 12 : 6-déoxy-4-O-méthyl-3-O-((5-méthyl-1H-pyrrol-2-yl) carbonyl)-alpha-L-mannopyranoside de (4-(diméthylamino)-8-méthyl-2-oxo-2H-1-benzopyran-7-yle**

F = 225°C

**EXEMPLE 13** : **6-déoxy-4-O-méthyl-3-0-((5-méthyl-1H-pyrrol-2-yl) carbonyl)-alpha-L-mannopyranoside de (8-méthyl-4-(4-morpholinyl)-2-oxo-2H-1-benzopyran-7-yle**

F = 190°C

**<u>EXEMPLE 14</u> : 6-déoxy-4-O-méthyl-3-O-((5-méthyl-1H-pyrrol-2-yl) carbonyl)-alpha-L-mannopyranoside de (8-méthyl-4-(4-méthyl-1-pipérazinyl)-2-oxo-2H-1-benzopyran-7-yle**

rf = 0,31 acétone-acétate d'éthyle-eau (5-4-1).

**<u>EXEMPLE 15</u> : 6-déoxy-4-O-méthyl-3-O-((5-méthyl-1H-pyrrol-2-yl) carbonyl)-alpha-L-mannopyranoside de l'iodure de 4,4-diméthyl-pipérazinium-1-yl)-8-méthyl-2-oxo-2H-1-benzopyran-7-yle**

En soumettant le produit de l'exemple précédent à l'action de l'iodure de méthyle, on a obtenu le produit recherché. rf = 0 acétone-acétate d'éthyle-eau (5-4-1).

**<u>EXEMPLE 16 :</u> Acide 5-méthyl-1H-pyrrole-2-carboxylique-3'-ester de 7-((6-déoxy-5-C-méthyl-4-O-méthyl-alpha-L-lyxo-hexopyranosyl) oxy)-8-méthyl-4-(3-méthyl-1-pipérazinyl)-2H-1-benzopyran-2-one**

En opérant comme précédemment, on a obtenu le produit recherché. F = 205°C.

**<u>EXEMPLE 17</u> : Acide 5-méthyl-1H-pyrrole-2-carboxylique-3'-ester de 7-((6-déoxy-5-C-méthyl-4-O-méthyl-alpha-L-lyxo-hexopyranosyl) oxy)-8-méthyl-4-(4-(2-piridinyl)-1-pipérazinyl)-2H-1-benzopyran-2-one**

En opérant comme précédemment, on a obtenu le produit recherché. F = 228~230°C.

**<u>EXEMPLE 18</u>: Acide 5-méthyl-1H-pyrrole-2-carboxylique-3'-ester de 7-((6-déoxy-5-C-méthyl-4-O-méthyl-alpha-L-lyxo-hexopyranosyl) oxy)-4-(4-dimétylamino)-1-pipéridinyl)-8-méthyl-2H-1-benzopyran-2-one**

On ajoute à la température ambiante dans une solution renfermant 2 mmole de 4-diméthylamino-1-pipéridine, dans 2,5 ml de THF, 0,8 mmole d'acide 5-méthyl-1H-pyrrole-2-carboxylique-3'-ester de 7-[(6-déoxy-5-C-méthyl-4-O-méthylalpha-L-lyxo-hexopyranosyl) oxy]-8-méthyl-4-[[(4-méthylphényl)sulfonyl]oxy]-2H-1-benzopyran-2-one. On agite pendant 2 heures. On verse dans une solution saturée de phosphate acide de sodium (1M). On extrait à l'acétate d'éthyle. On sèche, concentre et obtient 87 mg de produit que l'on additionne de 2 ml d'acétonitrile; On obtient un produit que l'on essore et sèche. On obtient ainsi 58 mg de produit. F = 190°C.

**<u>PREPARATION</u> : Acide 5-méthyl-1H-pyrrole-2-carboxylique-3'-ester de 7-[(6-déoxy-5-C-méthyl-4-O-méthyl-alpha-L-lyxo-hexopyranosyl) oxy]-8-méthyl-4-[[(4-méthylphényl)sulfonyl]oxy]-2H-1-benzopyran-2-one**

<u>STADE A</u> : 7-(hydroxy-8-méthyl-4-[[(4-méthylphényl)sulfonyl] oxy]-2H-1-benzopyran-2-one

On ajoute à 0°C, 3,5 g de chlorure de tosyle dans une solution renfermant 3,50 g de 4,7-dihydroxy 8-méthyl-2H-1-benzopyran-2-one et 25 ml de pyridine. On agite pendant 2 heures à 0°C, verse sur une solution aqueuse d'acide chlorhydrique 1,2N. On extrait à l'acétate d'éthyle, réunit les phases organiques, les sèche et concentre sous pression réduite. On chromatographie sur silice le produit obtenu en éluant avec le mélange acétone-chlorure de méthylène 5-95 et isole 3,88 g d'un produit que l'on empâte dans l'éther, et essore. On obtient le produit recherché. F = 206°C.

<u>STADE B</u> : Acide 5-méthyl-1H-pyrrole-2-carboxylique-3'-ester de 7-[(6-déoxy-5-C-méthyl-4-O-méthyl-alpha-L-lyxo-hexopyranosyl) oxy]-8-méthyl-4-[[(4-métylphényl)sulfonyl]oxy]-2H-1-benzopyran-2-one

On ajoute 1,4 g de triphénylphosphine et 886 µl de DEAD dans une solution de 1,87 g de produit du stade précédent, 2 g de 3,3-(5-méthyl)-1H-pyrrole-2-carboxylate) de 6-déoxy-5-C-méthyl-4-O-méthyl-L-lyxo-hexopyranose et 50 ml de chlorure de méthylène. On agite à la température ambiante pendant 1 heure, ajoute 0,5 équivalents de triphénylphosphine et DEAD, agite pendant 45 minutes, ajoute à nouveau 0,25 équivalents de triphénylphosphine et DEAD. On agite pendant 30 minutes, concentre. On filtre, chromatographie le produit obtenu sur silice en éluant avec le mélange chlorure de méthylène-acétate (95-5). On obtient 1,43 g du produit recherché. rf = 0,25.

**<u>EXEMPLE 19</u> : Acide 5-méthyl-1H-pyrrole-2-carboxylique-3'-ester de 7-((6-déoxy-5-C-méthyl-4-O-méthyl-alpha-L-lyxo-hexanopyranosyl) oxy)-4-(4-hydroxy-1-pipéridinyl)-8-méthyl-2H-1-benzopyran-2-one**

On ajoute 101,15 mg de 4-hydroxy 1-pipéridine dans une solution renfermant 216 mg d'acide 5-méthyl-1H-pyrrole-2-carboxylique-3'-ester de 7-[(6-déoxy-5-C-méthyl-4-O-méthyl-alpha-L-lyxo-hexopyranosyl) oxy]-8-méthyl-4-[[(4-métylphényl) sulfonyl]oxy]-2H-1-benzopyran-2-one et 5 ml de THF. On agite pendant 1 heure. On ajoute une

solution de phosphate acide de sodium 1M. On extrait à l'acétate d'éthyle et sèche. On concentre. On obtient un produit que l'on chromatographie sur silice en éluant avec le mélange chlorure de méthylène-acétone (6-4). On concentre et obtient 53 mg de produit recherché. F = 230°C.

**EXEMPLE 20 : (S) acide 5-méthyl-1H-pyrrole-2-carboxylique-3'-ester de 7-((6-déoxy-5-C-méthyl-4-O-méthyl-alpha-L-lyxo-hexopyranosyl) oxy)-4-[2-(hydroxyméthyl)-1-pyrrolidinyl]-8-méthyl-2H-1-benzopyran-2-one**

On introduit dans une solution de 607 mg d'acide 5-méthyl-1H-pyrrole-2-carboxylique-3'-ester de 7-((6-déoxy-5-C-méthyl-4-O-méthyl-alpha-L-lyxo-hexopyranosyl) oxy)-4-[[(4-méthylphényl)-sulfonyl]oxy]-2H-1-benzopyran-2-one et 5 ml de THF, 240 µl de 2-pyrrolidine méthanol. On agite à la température ambiante pendant 30 minutes. On verse dans 15 ml d'une solution 1M de $NaH_2PO_4$. On extrait à l'acétate d'éthyle, sèche et concentre. On obtient un produit que l'on chromatographie sur silice en éluant avec le mélange chlorure de méthylène-acétone (7-3). On concentre et obtient 173 mg de produit que l'on reprend dans l'acétonitrile. On essore, sèche et obtient 110 mg de produit recherché. F > 240°C.

**EXEMPLE 21 : Acide 5-méthyl-1H-pyrrole-2-carboxylique-3'-ester de 7-((6-déoxy-5-C-méthyl-4-O-méthyl-alpha-L-lyxo-hexopyranosyl) oxy)-4-(2-hydroxyméthyl)-4-morpholinyl]-8-méthyl-2H-1-benzopyran-2-one**

En opérant comme précédemment à partir d'acide 5-méthyl-1H-pyrrole-2-carboxylique-3'-ester de 7-[(6-déoxy-5-C-méthyl-4-O-méthyl-alpha-L-lyxo-hexopyranosyl) oxy]-8-méthyl-4-[[(4-(méthylphényl)-sulfonyl]oxy]-8-méthyl-2H-1-benzopyran-2-one et de 2-(hydroxyméthyl)-4-morpholine, on obtient le produit recherché. F = 260°C.

**EXEMPLE 22 : Acide 5-méthyl-1H-pyrrole-2-carboxylique-3'-ester de 7-((6-déoxy-5-C-méthyl-4-O-méthyl-alpha-L-lyxo-hexopyranosyl) oxy)-8-méthyl-4-(méthylamino)-2H-1-benzopyran-2-one**

En opérant comme précédemment à partir de 500 mg d'acide 5-méthyl-1H-pyrrole-2-carboxylique-3'-ester de 7-[(6-déoxy-5-C-méthyl-4-O-méthyl-alpha-L-lyxo-hexopyranosyl) oxy]-8-méthyl- 4-[[(4-méthylphényl)sulfonyl]oxy]-2H-1-benzopyran-2-one et de la méthylamine, on a obtenu le produit recherché. rf = 0,27 chlorure de méthylène-isopropanol (9-1).

**EXEMPLE 23: Acide 5-méthyl-1H-pyrrole-2-carboxylique-3'-ester de 7-((6-déoxy-5-C-méthyl-4-O-méthyl-alpha-L-lyxo-hexopyranosyl) oxy)-4-(2-((diméthylamino)méthyl)-4-morpholinyl]-8-méthyl-2H-1-benzopyran-2-one**

En opérant comme précédemment, à partir de 2-(diméthylamino)méthyl-4-morpholine et d'acide 5-méthyl-1H-pyrrole-2-carboxylique-3'-ester de 7-[(6-déoxy-5-C-méthyl-4-O-méthyl-alpha-L-lyxo-hexopyranosyl) oxy]-8-méthyl- 4-[[(4-méthyl phényl)sulfonyl]oxy]-2H-1-benzopyran-2-one, on a obtenu le produit recherché. rf = 0,18 chlorure de méthylène-éthanol (8-2).

En opérant comme précédemment, on a obtenu les produits suivants :

**EXEMPLE 24 : Acide 5-méthyl-1H-pyrrole-2-carboxylique-3'-ester de 7-((6-déoxy-5-C-méthyl-4-O-méthyl-alpha-L-lyxo-hexopyranosyl) oxy)-4-(3,5-diméthyl-1-pipéridinyl)-8-méthyl-2H-1-benzopyran-2-one**

F = 260~262°C.

**EXEMPLE 25: acide 5-méthyl-1H-pyrrole-2-carboxylique-3'-ester de 7-[(6-déoxy-5-C-méthyl-4-O-méthyl-alpha-L-lyxo-hexopyranosyl) oxy]-4-(4-(2-hydroxyéthyl)1-pipérazinyl)-8-méthyl-2H-1-benzopyran-2-one**

F = 230~232°C.

**EXEMPLE 26 : (trans) acide 5-méthyl-1H-pyrrole-2-carboxylique-3'-ester de 7-[(6-déoxy-5-C-méthyl-4-O-méthyl-alpha-L-lyxo-hexopyranosyl)oxy]-4-(2,5-diméthyl-1-pipérazinyl)-8-méthyl-2H-1-benzopyran-2-one (mélange de diastéréoisomères)**

rf = 0,1 chlorure de méthylène-éthanol (90-10).

EXEMPLES DE COMPOSITIONS PHARMACEUTIOUES

On a préparé des comprimsés renfermant :

| | |
|---|---|
| **Produit de l'exemple 1**<br>Excipient q.s.p. | 150 mg<br>1 g |
| Détail de l'excipient : | amidon, talc, stéarate de magnésium. |
| **Produit de l'exemple 2**<br>Excipient q.s.p. | 150 mg<br>1 g |
| Détail de l'excipient : | amidon, talc, stéarate de magnésium. |

On a également préparé des solutions injectables à partir des salifiés.

**ETUDE PHARMACOLOGIOUE DES PRODUITS DE L'INVENTION**

A - Méthode des dilutions en milieu liquide

On a préparé une série de tubes dans lesquels on répartit une même quantité de milieu nutritif stérile. On distribue dans chaque tube des quantités croissantes du produit à étudier, puis chaque tube est ensemencé avec une souche bactérienne. Après incubation de 24 heures à l'étuve à 37°C, l'inhibition de la croissance est appréciée par transillumination ce qui permet de déterminer les concentrations minimales inhibitrices (C.M.I.) exprimées en microgrammes/$cm^3$. Sur les souches suivantes :

| | |
|---|---|
| S. aureus | 011HT3 |
| S. aureus | 011UC4 |
| S. aureus | 011HT28 |
| S. epidermidis | 012GO20 |
| S. aureus | 011CB20 |
| S. aureus | 011HT26 |
| S. epidermidis | 012GO39 |
| S. epidermidis | 012HI1 |
| S. pyogenes | 02A1UC1 |

Les résultats suivants ont été obtenus :

$$0{,}04 < CMI < 5$$

B - Inhibition de la gyrase B

Les produits sont des inhibiteurs de gyrase B ; la dose à 50 % du surenroulement de l'ADN est inférieure à 5 µg/ml.

**Revendications**

**1.** Les composés de formule (I) :

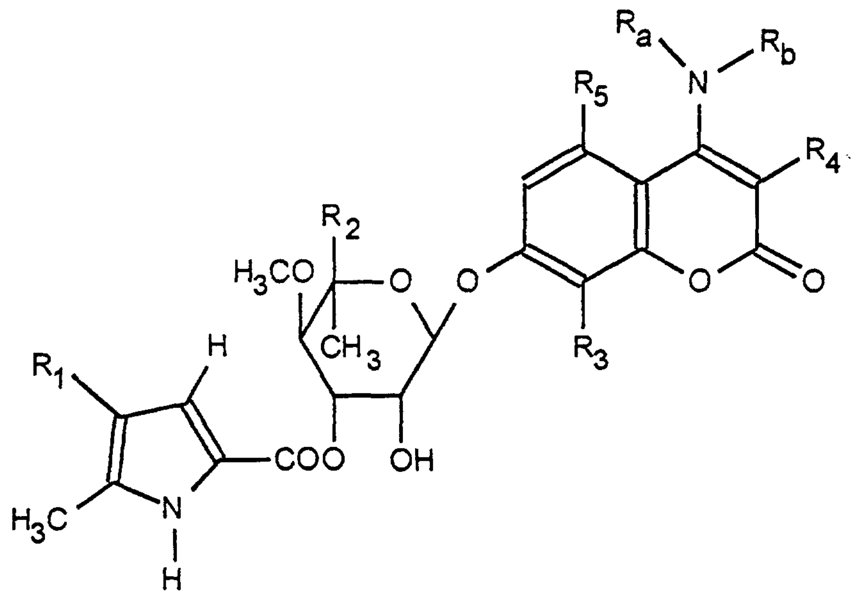

(I)

dans lesquels :

- $R_1$ représente un atome d'hydrogène ou d'halogène,
- $R_2$ représente un atome d'hydrogène ou un radical alkyle renfermant jusqu'à 4 atomes de carbone,
- $R_3$ représente un radical alkyle renfermant jusqu'à 4 atomes de carbone, ou un atome d'halogène,
- $R_4$ représente un atome d'hydrogène, un atome d'halogène, un radical alkyle, alkényle ou alkynyle renfermant jusqu'à 12 atomes de carbone, linéaire, ramifié ou cyclique,
- $R_5$ représente un atome d'hydrogène, un radical OH ou O-alkyle, renfermant jusqu'à 12 atomes de carbone,

$R_a$ et $R_b$ identiques ou différents représentent un atome d'hydrogène, un radical alkyle renfermant jusqu'à 4 atomes de carbone, ou bien $R_a$ et $R_b$ forment avec l'atome d'azote auquel ils sont liés un hétérocycle mono ou polycyclique, renfermant éventuellement un autre hétéroatome choisi parmi l'azote, le soufre ou l'oxygène, et pouvant être éventuellement substitué, ou bien $R_a$ et/ou $R_b$ représentent un radical :

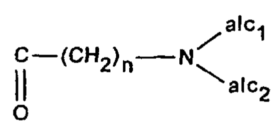

dans lequel n est un nombre entier variant de 0 à 6, $alc_1$ et $alc_2$ représentent un radical alkyle renfermant jusqu'à 8 atomes de carbone et leurs sels sous toutes leurs formes stéréoisomères possibles ainsi que leurs mélanges.

**2.** Les composés de formule (I) définis à la revendication 1, dans lesquels $R_1$ représente un atome d'hydrogène ou de fluor.

**3.** Les composés de formule (I) définis à la revendication 1 ou 2, dans lesquels $R_2$ représente un radical méthyle ou un atome d'hydrogène.

**4.** Les composés de formule (I) dans lesquels $R_1$, $R_2$, $R_4$, $R_5$, $R_a$ et $R_b$ sont tels que définis à l'une quelconque des revendications 1 à 3, et dans lesquels $R_3$ représente un radical méthyle,ou un atome de chlore.

**5.** Les composés de formule (I) définis à l'une quelconque des revendications 1 à 4, dans lesquels $R_4$ représente un atome d'hydrogène ou de chlore.

**6.** Les composés de formule (I) définis à l'une quelconque des revendications 1 à 5 dans lesquel $R_5$ représente un

atome d'hydrogène.

**7.** Les composés de formule (I) définis à l'une quelconque des revendications 1 à 6, dans lesquels $R_a$ et $R_b$ représentent un radical alkyle renfermant jusqu'à 4 atomes de carbone.

**8.** Les composés de formule (I) définis à la revendication 7 dans lesquels $R_a$ et $R_b$ représentent un radical méthyle.

**9.** Les composés de formule (I) définis à l'une quelconque des revendications 1 à 6, dans lesquels $R_a$ et $R_b$ forment avec l'atome d'azote auquel ils sont liés un radical hétérocyclique renfermant éventuellement un autre hétéroatome, et pouvant être éventuellement substitué.

**10.** Les composés de formule (I) définis à la revendication 9 dans lesquels $R_a$ et $R_b$ forment ensemble un radical

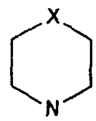

dans lequel X représente un atome d'oxygène ou un radical NH, ou $NCH_3$, le radical hétérocyclique ainsi formé pouvant être éventuellement substitué.

**11.** Les composés de formule (I) définis à la revendication 1 dont les noms suivent :

- 6-déoxy-5-C-méthyl-4-O-méthyl-3-O-((5-méthyl-1H-pyrrol-2-yl) carbonyl)-alpha-L-lyxo-hexopyranoside de (4-(diméthylamino)-8-méthyl-2-oxo-2H-1-benzopyran-7-yle),
- 6-déoxy-5-C-méthyl-4-O-méthyl-3-O-((5-méthyl-1H-pyrrol-2-yl) carbonyl)-alpha-L-lyxo-hexopyranoside de (8-méthyl-4-(4-méthyl-1-pipérazinyl)-2-oxo-2H-1-benzopyran-7-yle).

**12.** A titre de médicaments antibiotiques, les composés de formule (I) définis à l'une quelconque des revendications 1 à 10.

**13.** A titre de médicaments antibiotiques, les composés de formule (I) définis à la revendication 11.

**14.** Les compositions pharmaceutiques renfermant comme principe actif au moins un médicament antibiotique défini à l'une des revendications 12 ou 13.

**15.** Procédé de préparation des composés de formule (I) définis à l'une quelconque des revendications 1 à 11, **caractérisé en ce que** l'on soumet un composé de formule (II) :

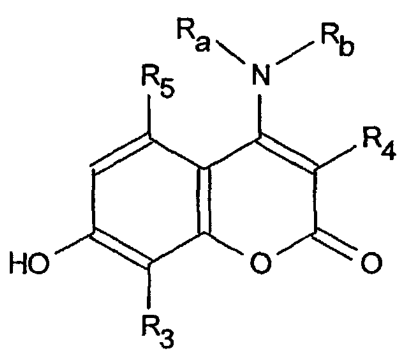

(II)

dans lequel $R_3$, $R_4$, $R_5$, $R_a$ et $R_b$ conservent leur signification précédente, à l'action d'un composé de formule (III) :

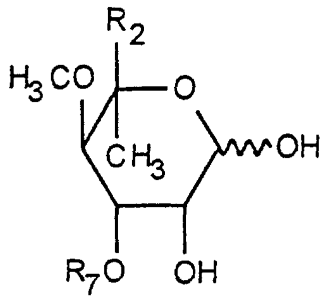

(III)

dans lequel $R_2$ conserve sa signification précédente et $R_7$ représente un radical

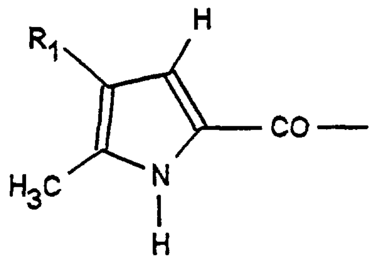

dans lequel $R_1$ conserve sa signification précédente, pour obtenir le composé de formule (I) correspondant que l'on modifie si désiré.

**16.** Variante du procédé de la revendication 15, **caractérisée en ce que** l'on soumet un composé de formule (IV) :

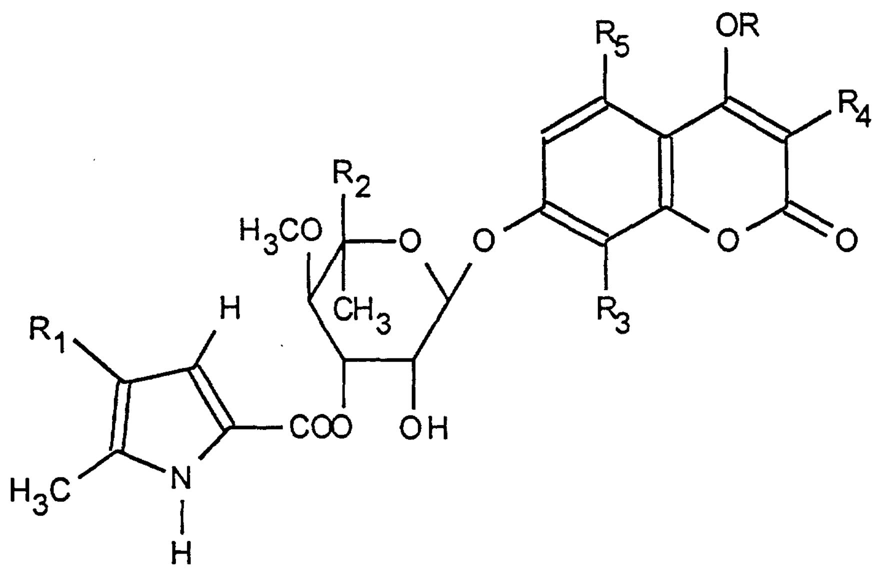

(IV)

dans lequel $R_1$, $R_2$, $R_3$, $R_4$ et $R_5$ conservent leur signification précédente et R représente un groupe partant à l'action amine

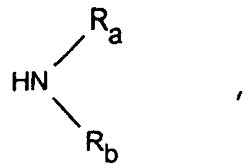

dans laquelle $R_a$ et $R_b$ conservent leur signification précédente pour obtenir le composé de formule (I) correspondant que l'on modifie si désiré.

**17.** A titre de produits chimiques nouveaux, les composés de formules (II) et (IV), tels que définis aux revendications 15 et 16, à l'exception du 4-amino 7-hydroxy-8-methylbenzopyran-2-one, du 4-amino-8-ethyl-7-hydroxycoumarin et du 4-hydroxy-7α-3-O-[5'-methyl-2'-carbonyl-pyrrole]-noviosyloxy-8-methyl-coumarin.

**Patentansprüche**

**1.** Verbindungen der Formel (I)

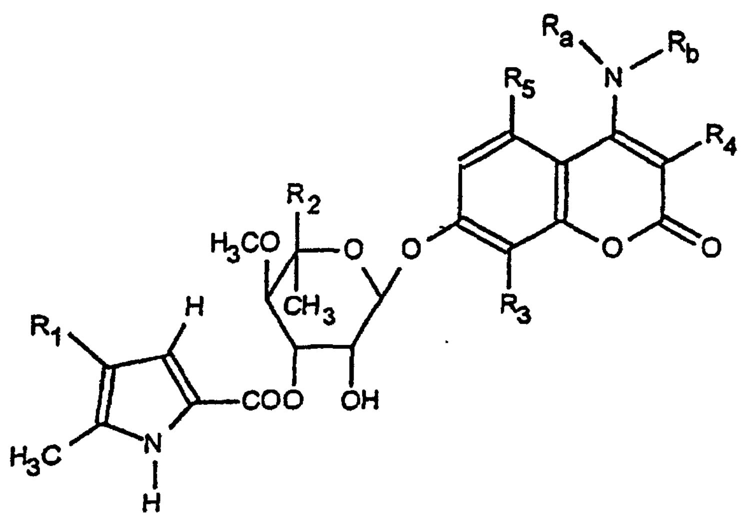

(I)

in der

- $R_1$ ein Wasserstoffatom oder ein Halogenatom darstellt,
- $R_2$ ein Wasserstoffatom oder einen Rest Alkyl mit bis zu 4 Kohlenstoffatomen darstellt,
- $R_3$ einen Rest Alkyl mit bis zu 4 Kohlenstoffatomen oder ein Halogenatom darstellt,
- $R_4$ ein Wasserstoffatom, ein Halogenatom, einen linearen, verzweigten oder cyclischen Rest Alkyl, Alkenyl oder Alkinyl mit bis zu 12 Kohlenstoffatomen darstellt,
- $R_5$ ein Wasserstoffatom, einen Rest OH oder O-alkyl mit bis zu 12 Kohlenstoffatomen darstellt,
- $R_a$ und $R_b$, gleich oder verschieden, ein Wasserstoffatom, einen Rest Alkyl mit bis zu 4 Kohlenstoffatomen darstellen, oder $R_a$ und $R_b$ auch zusammen mit dem Stickstoffatom, an das sie gebunden sind, einen mono- oder polycyclischen Heterocyclus mit gegebenenfalls einem weiteren Heteroatom bilden, ausgewählt unter Stickstoff, Schwefel oder Sauerstoff, der gegebenenfalls substituiert sein kann, oder auch $R_a$ und/oder $R_b$

worin n eine ganze Zahl von 0 bis 6 ist, $alc_1$ und $alc_2$ einen Rest Alkyl mit bis zu 8 Kohlenstoffatomen darstellen, und ihre Salze in allen ihren möglichen stereoisomeren Formen sowie ihre Mischungen.

**2.** Verbindungen der Formel (I) wie in Anspruch 1 definiert, worin $R_1$ ein Wasserstoffatom oder ein Fluoratom darstellt.

**3.** Verbindungen der Formel (I) wie in Anspruch 1 oder 2 definiert, worin $R_2$ einen Rest Methyl oder ein Wasserstoffatom darstellt.

**4.** Verbindungen der Formel (I), worin $R_1$, $R_2$, $R_4$, $R_5$, $R_a$ und $R_b$ wie in irgendeinem der Ansprüche 1 bis 3 definiert sind und worin $R_3$ einen Rest Methyl oder ein Chloratom darstellt.

**5.** Verbindungen der Formel (I) wie in irgendeinem der Ansprüche 1 bis 4 definiert, worin $R_4$ ein Wasserstoffatom oder ein Chloratom darstellt.

**6.** Verbindungen der Formel (I) wie in irgendeinem der Ansprüche 1 bis 5 definiert, worin $R_5$ ein Wasserstoffatom darstellt.

**7.** Verbindungen der Formel (I) wie in irgendeinem der Ansprüche 1 bis 6 definiert, worin $R_a$ und $R_b$ einen Rest Alkyl mit bis zu 4 Kohlenstoffatomen darstellen.

**8.** Verbindungen der Formel (I) wie in Anspruch 7 definiert, worin $R_a$ und $R_b$ einen Rest Methyl darstellen.

**9.** Verbindungen der Formel (I) wie in irgendeinem der Ansprüche 1 bis 6 definiert, worin $R_a$ und $R_b$ zusammen mit dem Stickstoffatom, an das sie gebunden sind, einen heterocyclischen Rest bilden, der gegebenenfalls ein weiteres Heteroatom umfaßt, und der gegebenenfalls substituiert sein kann.

**10.** Verbindungen der Formel (I) wie in Anspruch 9 definiert, worin $R_a$ und $R_b$ zusammen einen Rest

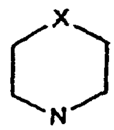

bilden, worin X ein Sauerstoffatom oder einen Rest NH oder $NCH_3$ darstellt, wobei der auf diese Weise gebildete heterocyclische Rest gegebenenfalls substituiert sein kann.

**11.** Verbindungen der Formel (I) wie in Anspruch 1 definiert, mit den folgenden Namen:

- 6-Deoxy-5-C-methyl-4-O-methyl-3-O-[(5-methyl-1H-pyrrol-2-yl)-carbonyl]-alpha-L-lyxo-hexopyranosid von [4-(Dimethylamino)-8-methyl-2-oxo-2H-1-benzopyran-7-yl],
- 6-Deoxy-5-C-methyl-4-O-methyl-3-O-[(5-methyl-1H-pyrrol-2-yl)-carbonyl]-alpha-L-lyxo-hexopyranosid von [8-Methyl-4-(4-methyl-1-piperazinyl)-2-oxo-2H-1-benzopyran-7-yl].

**12.** Als antibiotische Arzneimittel die Verbindungen der Formel (I) wie in irgendeinem der Ansprüche 1 bis 10 definiert.

**13.** Als antibiotische Arzneimittel die Verbindungen der Formel (I) wie in Anspruch 11 definiert.

**14.** Pharmazeutische Zusammensetzungen, umfassend als Wirkstoff mindestens ein antibiotisches Arzneimittel wie in einem der Ansprüche 12 oder 13 definiert.

**15.** Verfahren zur Herstellung der Verbindungen der Formel (I) wie in irgendeinem der Ansprüche 1 bis 11 definiert, **dadurch gekennzeichnet, daß** man eine Verbindung der Formel (II)

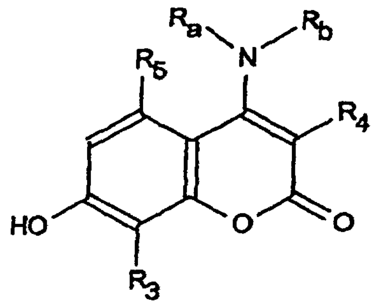

(II)

in der $R_3$, $R_4$, $R_5$, $R_a$ und $R_b$ ihre vorstehende Bedeutung beibehalten, der Einwirkung einer Verbindung der Formel (III)

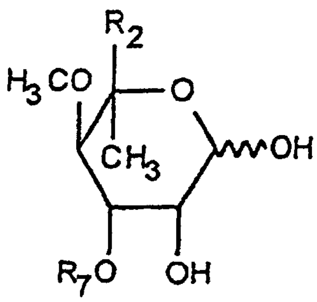

(III)

unterzieht,
in der $R_2$ seine vorstehende Bedeutung beibehält und $R_7$ einen Rest der Formel

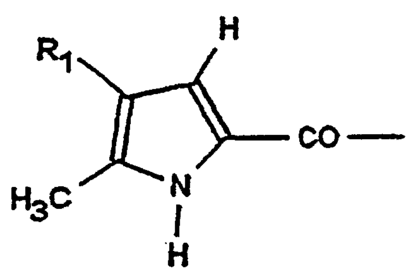

darstellt, in der $R_1$ seine vorstehende Bedeutung beibehält, um die entsprechende Verbindung der Formel (I) zu erhalten, die man, wenn erwünscht, modifiziert.

**16.** Variante des Verfahrens nach Anspruch 15, **dadurch gekennzeichnet, daß** man eine Verbindung der Formel (IV)

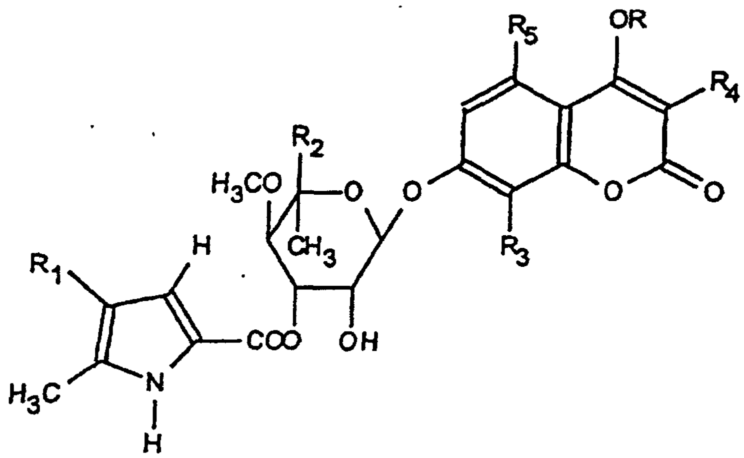

(IV)

in der

$R_1$, $R_2$, $R_3$, $R_4$ und $R_5$ ihre vorstehende Bedeutung beibehalten und R eine Abgangsgruppe darstellt, der Einwirkung eines Amins

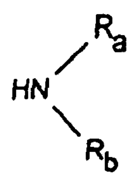

unterzieht, worin $R_a$ und $R_b$ ihre vorstehende Bedeutung beibehalten, um die Verbindung der Formel (I) zu erhalten, die man, wenn erwünscht, modifiziert.

**17.** Als neue chemische Produkte die Verbindungen der Formeln (II) und (IV) wie in Anspruch 15 und 16 definiert, mit Ausnahme von
4-Amino-7-hydroxy-8-methyl-benzopyran-2-on,
4-Amino-8-ethyl-7-hydroxy-cumarin und
4-Hydroxy-7α-3-O-[5'-methyl-2'-carbonyl-pyrrol]-noviosyloxy-8-methyl-cumarin.

**Claims**

**1.** The compounds of formula (I):

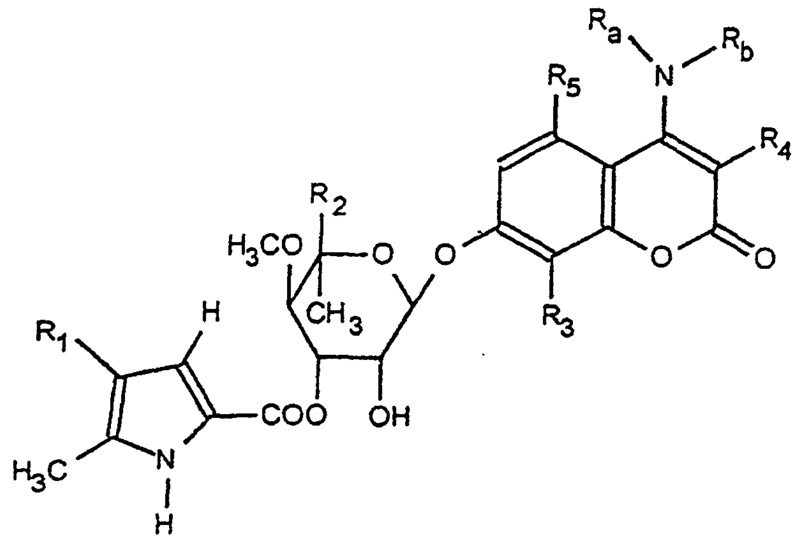

(I)

in which:

- $R_1$ represents a hydrogen or halogen atom,
- $R_2$ represents a hydrogen atom or an alkyl radical containing up to 4 carbon atoms,
- $R_3$ represents an alkyl radical containing up to 4 carbon atoms, or a halogen atom,
- $R_4$ represents a hydrogen atom, a halogen atom, or a linear, branched or cyclic alkyl, alkenyl or alkynyl radical containing up to 12 carbon atoms,
- $R_5$ represents a hydrogen atom, a radical OH or a radical O-alkyl containing up to 12 carbon atoms,

$R_a$ and $R_b$, which may be identical or different, represent a hydrogen atom, or an alkyl radical containing up to 4 carbon atoms, or else $R_a$ and $R_b$ form, with the nitrogen atom to which they are attached, a monocyclic or polycyclic heterocycle optionally containing another hetero atom chosen from nitrogen, sulphur or oxygen, and which may optionally be substituted, or else $R_a$ and/or $R_b$ represent a radical:

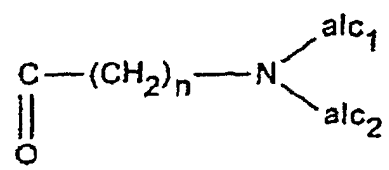

in which n is an integer ranging from 0 to 6, $alc_1$ and $alc_2$ represent an alkyl radical containing up to 8 carbon atoms, and their salts in any of their possible stereoisomeric forms and also their mixtures.

**2.** The compounds of formula (I) defined in Claim 1, in which $R_1$ represents a hydrogen or fluorine atom.

**3.** The compounds of formula (I) defined in Claim 1 or 2, in which $R_2$ represents a methyl radical or a hydrogen atom.

**4.** The compounds of formula (I) in which $R_1$, $R_2$, $R_4$, $R_5$, $R_a$ and $R_b$ are as defined in any one of Claims 1 to 3, and in which $R_3$ represents a methyl radical or a chlorine atom.

**5.** The compounds of formula (I) defined in any one of Claims 1 to 4, in which $R_4$ represents a hydrogen or chlorine atom.

**6.** The compounds of formula (I) defined in any one of Claims 1 to 5, in which $R_5$ represents a hydrogen atom.

**7.** The compounds of formula (I) defined in any one of Claims 1 to 6, in which $R_a$ and $R_b$ represent an alkyl radical containing up to 4 carbon atoms.

**8.** The compounds of formula (I) defined in Claim 7, in which $R_a$ and $R_b$ represent a methyl radical.

**9.** The compounds of formula (I) defined in any one of Claims 1 to 6, in which $R_a$ and $R_b$ form, with the nitrogen atom to which they are attached, a heterocyclic radical optionally containing another hetero atom, and which may be optionally substituted.

**10.** The compounds of formula (I) defined in Claim 9, in which $R_a$ and $R_b$ together form a radical

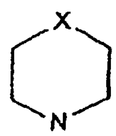

in which X represents an oxygen atom or a radical NH or $NCH_3$, it being possible for the heterocyclic radical thus formed to be optionally substituted.

**11.** The compounds of formula (I) defined in Claim 1, the names of which follow:

- (4-(dimethylamino)-8-methyl-2-oxo-2H-1-benzopyran-7-yl) 6-deoxy-5-C-methyl-4-O-methyl-3-O-((5-methyl-1H-pyrrol-2-yl)carbonyl)-alpha-L-lyxohexopyranoside,
- (8-methyl-4-(4-methyl-1-piperazinyl)-2-oxo-2H-1-benzopyran-7-yl) 6-deoxy-5-C-methyl-4-O-methyl-3-O-((5-methyl-1H-pyrrol-2-yl)carbonyl)-alpha-L-lyxohexopyranoside.

**12.** The compounds of formula (I) defined in any one of Claims 1 to 10, as antibiotic medicinal products.

**13.** The compounds of formula (I) defined in Claim 11, as antibiotic medicinal products.

**14.** The pharmaceutical compositions containing, as active principle, at least one antibiotic medicinal product defined in either of Claims 12 and 13.

**15.** Process for preparing the compounds of formula (I) defined in any one of Claims 1 to 11, **characterized in that** a compound of formula (II):

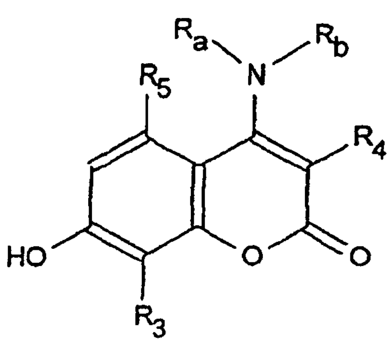

(II)

in which $R_3$, $R_4$, $R_5$, $R_a$ and $R_b$ conserve their meaning above, is subjected to the action of a compound of formula (III):

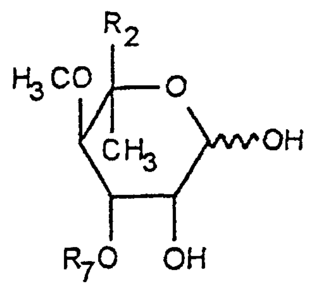

(III)

in which $R_2$ conserves its meaning above and $R_7$ represents a radical

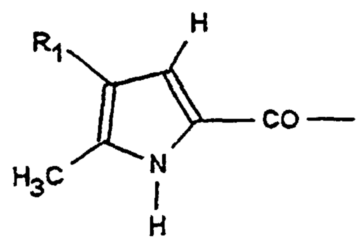

in which $R_1$ conserves its meaning above, so as to obtain the corresponding compound of formula (I), which is modified if desired.

**16.** Variant of the process of Claim 15, **characterized in that** a compound of formula (IV):

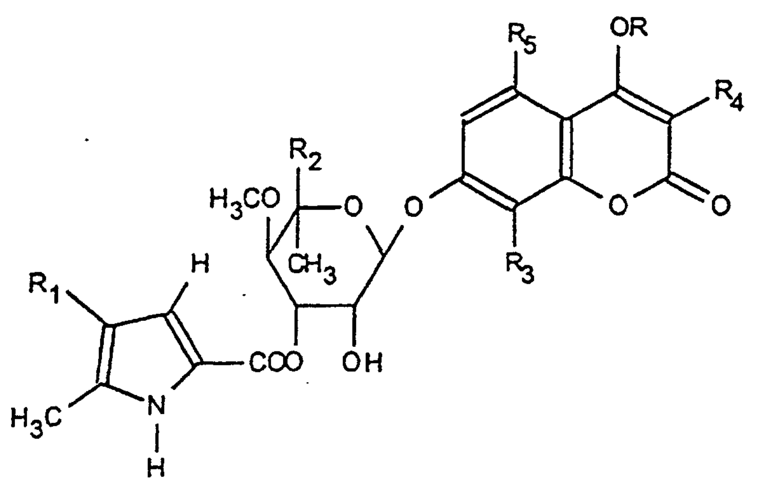

(IV)

in which $R_1$, $R_2$, $R_3$, $R_4$ and $R_5$ conserve their meaning above and R represents a leaving group, is subjected to the action of an amine

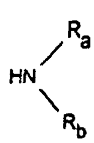

,

in which $R_a$ and $R_b$ conserve their meaning above, so as to obtain the corresponding compound of formula (I), which is modified if desired.

**17.** As novel chemical products, the compounds of formulae (II) and (IV) , as defined in Claims 15 and 16, with the exception of 4-amino-7-hydroxy-8-methylbenzopyran-2-one, of 4-amino-8-ethyl-7-hydroxycoumarin and of 4-hydroxy-7α-3-O-[5'-methyl-2'-carbonylpyrrole]-noviosyloxy-8-methylcoumarin.